# EUROPEAN PATENT SPECIFICATION

(11) **EP 3 122 764 B1**
(45) Date of publication and mention of the grant of the patent: **16.01.2019**
(21) Application number: 15720489.2
(22) Date of filing: 25.03.2015
(51) Int. Cl.: C07K 7/08, C07K 14/47, C07K 7/54

(54) **CYCLIC APELIN ANALOGS**
CYCLISCHE APELINANALOGA
ANALOGUES D'APELINE CYCLIQUE

(30) Priority: 25.03.2014 EP 14161533
(43) Date of publication of application: 01.02.2017
(73) Proprietor: LanthioPep B.V., 9727 DL Groningen (NL)
(72) Inventor: RINK, Rick, NL-9727 DL Groningen (NL)
(74) Representative: Spiller, Stephan
(86) International application number: PCT/NL2015/050191
(87) International publication number: WO 2015/147641

(56) References cited:
- EP-A1- 2 405 008
- US-A1- 2013 196 899
- BOSMA TJIBBE ET AL: "Bacterial Display and Screening of Posttranslationally Thioether-Stabilized Peptides", APPLIED AND ENVIRONMENTAL MICROBIOLOGY, AMERICAN SOCIETY FOR MICROBIOLOGY, US, vol. 77, no. 19, 1 October 2011 (2011-10-01), pages 6794-6801, XP009153580, ISSN: 0099-2240
- JURI HAMADA ET AL: "Evaluation of novel cyclic analogues of apelin.", INTERNATIONAL JOURNAL OF MOLECULAR MEDICINE, vol. 22, no. 4, 1 October 2008 (2008-10-01), pages 547-552, XP055072884, ISSN: 1107-3756

## Description

The invention relates to the field of pharmacology. In particular, it relates to novel analogs of apelin, and to the therapeutic uses thereof.

The apelin gene encodes a pre-proprotein of 77 amino acids that is processed to generate bioactive peptides consisting of 36, 17, or 13 amino acids (apelin-36, apelin-17, and apelin-13, respectively). The two major isoforms are apelin-13 and apelin 36.

The apelin receptor, APJ, is a G-protein coupled receptor (GPCR). Typical apelin-stimulated APJ signaling involves activation of pertussis toxin sensitive G-proteins (Gi or Go) leading to reduced cAMP production as well as activation of phospholipase C, protein kinase C and the extracellular signal-regulated kinases 1 and 2. See O'Carroll et al. (J. of Endocrinology (2013) 219, R13-R35) for a recent overview on apelin signaling. In addition, stimulation of APJ with apelin leads to recruitment of β-arrestins (Lee et al. Biochem Biophys Res Commun. 2010;395:185-189). As reviewed by Ma and Pei (J. Cell Science (2007) 120, 213-218) the arrestins form a group of scaffold proteins that not only regulate receptor desensitization and internalization, but also activate alternative G-protein-independent signaling pathways downstream of GPCRs. For example, in response to activation of certain GPCRs, β-arrestins translocate from the cytoplasm to the nucleus and associate with transcription cofactors such as p300 and cAMP-response element-binding protein (CREB) at the promoters of target genes to promote transcription. They also interact with regulators of transcription factors, such as IκBα and MDM2, in the cytoplasm and regulate transcription indirectly. This β-arrestin-mediated regulation of transcription appears to play important roles in cell growth, apoptosis and modulation of immune functions.

Apelin and APJ are widely expressed in homogenates from animal organs in a pattern shared with angiotensinogen and the angiotensin receptor. Apelin is widely distributed in the CNS and periphery, especially in the heart, kidney, lung and mammary glands. Apelin-like immunoreactivity was detected in the adipocytes, gastric mucosa, endothelia and Kupffer cells in the liver.

Apelin-13 is the endogenous ligand of the APJ receptor, activating this G protein-coupled receptor with an EC50 value of 0.37 nM (the EC50 values for apelin-17 and apelin-36 are 2.5 and 20 nM, respectively). It acts primarily in the periphery and central nervous system, playing important roles in regulating cardiovascular function, fluid homeostasis, hypertension, and insulin sensitivity. Unlike apelin-36, apelin-13 poorly blocks the entry of human immunodeficiency virus into cells.

Binding of apelin-13 and 36 to APJ inhibits forskolin-induced cAMP production and mediates Ras-independent activation of extracellular regulated kinases (ERK's). The binding of apelin-13 and -36 both lead to internalization of APJ, which is recycled to the outer cell surface (Zhou, N et al., 2003 APJ. Virology 307, 22-36).

Exogenous apelin administration alters cardiovascular function, blood pressure, body temperature, body fluid and behaviors involved in food intake and water intake. Several human cardiovascular diseases are accompanied by changes in apelin and/or APJ expression in cardiovascular tissues. In animal studies, apelin exerts vasodilation and positive inotropic actions. Apelin activates endothelial nitric oxide synthase and consequently stimulates nitric oxide release from the vascular endothelial cells. Apelin also ameliorated prognosis for heart function under pathological condition, such as experimental ischemia and reperfusion injury (Lee, D. K., George, S. R. and O'Dowd, B. F. (2006) Trends in Pharmacological Sciences 27, 190-194). However, these cardiovascular effects are short lived due to the short circulating life of the Apelin peptide.

Attempts to improve the half life of apelins are known in the art. For example, WO2012/125408 discloses a pegylated Apelin that comprises one or more polyethylene glycol (PEG) molecules operably linked to at least one amino acid residue in the N-terminal of an Apelin. The pegylated Apelin was found to have a prolonged circulating life and inotropic effect, relative to a non-pegylated Apelin. Another approach to improve the half life of apelins involves the generation of cyclic variants. For example, large cyclic variants (1 to 12; 1 to 7; and 7 to 12) of apelin 12 have been made which exhibited dose-dependent inhibitory effects against forskolin-induced cyclic adenosine monophosphate (cAMP) accumulation, and the maximal effects were almost abolished by pertussis toxin (PTx) treatment (Hamada, J. (2008) International Journal of Molecular Medicine. 22, 547-552). However, all cyclic analogs had several orders of magnitude reduced activity compared to pE-apelin13

WO2013/111110 discloses synthetic apelin mimetics for the treatment of heart failure. Variants include cyclic structures wherein amino acid side chains are linked together via either a monosulfide, a disulfide or an amide bond.

Accordingly, there exists a need for improved Apelin analogs so as to improve their circulating life and other beneficial characteristics. In particular, it would be desirable that the analog has an increased half-life combined with a differential biological activity, e.g. that it can be used to selectively modulate different pathways activated via the APJ receptor. For example, it would be of interest to have analogs that exhibit significant (agonistic) signaling via cAMP activity but which are less potent than the endogenous agonists at inducing β-arrestin recruitment, APJ receptor internalization and/or downregulation, such that biological activity is enhanced. Conversely, in view of β-arrestin-mediated regulation of transcription and its role in cell growth, apoptosis and modulation of immune functions, variants that are more potent stimuli of arrestin recruitment provide interesting biased agonists as well. In particular, effects on beta arrestin recruitment and Gi/0 stimulation may lead to different effects and / or effects with different kinetics in the cell. Arrestin does not seem to act via the Akt route, whereas Gi/0 does. G-protein-dependent ERK activation appears to be rapid and transient and can be blocked by pertussis toxin or PKA inhibitors. By contrast, beta-arrestin dependent ERK activation is slower in onset and more sustained in duration, and it is sensitive to depletion of beta-arrestins but not Gi/PKA inhibition or loss of Gs (Lan Ma 2006).Gi/0 stimulation of MAPK and arrestin-scaffold effects on MAPK differently affect distribution of ERK in cytoplasma/nucleus with consequences for cell fate.

It was surprisingly found that at least some of the above goals are met by the provision of novel cyclic apelin compounds that are characterized by a thioether bridge spanning 2, 3, 4, or 5 amino acid residues to yield a spatially constrained polypeptide having biological activity. For example, introduction of a thioether bridge into apelin resulted in a 2- to 8-fold increase in stability in plasma, while signaling activity was maintained. Moreover, a number of interesting variants were identified which exhibit either a significant increase or decrease in signaling via cAMP relative to arrestin as compared to native apelin.

Accordingly, the invention provides
an apelin analog consisting of a polypeptide of the general formula X1-X2-X3-X4-X5-X6-X7-X8-X9-X10-X11-X12-X13-X14, comprising one Lanthionine bridge of the structure Ala-S-Ala or one methylLanthionine bridge of the structure Abu-S-Ala or Ala-S-Abu, wherein
X1 is absent
X2 pGlu;
X3 is Arg
X4 is Pro, Pro-(me)Lan,
X5 is Arg, (me)Lan, Arg-(me)Lan,
X6 is Leu, (me)Lan, Leu-(me)Lan,
X7 is Ala, (me)Lan,
X8 is His, (me)Lan,
X9 is Lys, (me)Lan, Lys-(me)Lan,
X10 is Gly, (me)Lan,
X11 is Pro, (me)Lan,
X12 is Met, (me)Lan, Met-(me)Lan
X13 is either absent or selected from Pro, Dhb, (me)Lan,
X14 is the C-terminus and is either absent or is Phe unless X13 is absent;
in which Dhb means dehydrobutyrine (me)Lan means Lan or meLan, wherein Lan denotes the N- or C-terminal half of a Lanthionine (Ala-S-Ala) and meLan denotes the N- or C-terminal half of a methylLanthionine (Abu-S-Ala or Ala-S-Abu);
with the proviso that:
(i) the analog contains up to two Ala residues;
(ii) the sequence X4 through X13 contains one pair of meLan or one pair of Lan which together form a (methyl)Lanthionine bridge; and
(iii) wherein said (methyl)Lanthionine bridge is of the size i, i+3; i, i+4, i, i+5, or i, i+6, preferably i, i+3 or i, i+4; wherein "i " denotes the residue (e.g. X4) of the N-terminal amino acid involved in thioether-bridge formation and wherein "i+..." denotes the residue (e.g. X7 in case of i+3) of the C-terminal amino acid involved in thioether-bridge formation;
or an amide, an ester or a salt thereof.

It was found that a (methyl)lanthionine bridge structure of the size i, i+1 provides little proteolytic protection while the bridge i, i+2 is difficult to make. I, i+5 and i, i+6 are less ideal substrate for cyclase activity, but confer better proteolytic resistance. In a preferred embodiment said (methyl)Lanthionine bridge is of the size i, i+3 or i, i+4, such that there are either two or three residues under the ring.

This invention relates to (methyl)lanthionine variants of apelin in which the thioether-bridge is formed by either (i) replacement of two amino acids of the natural sequence; (ii) replacement of one amino acid of the natural sequence and insertion of one amino acid at either the N- or C-terminal side of the replacement; or (iii) two inserted amino acids.

A lanthionine-containing variant is characterized by the structure Ala-S-Ala, which is formed by two "Lan" moieties present (either by replacement or insertion) in the stretch X4-X13. A methyllanthionine-containing variant is characterized by the structure Abu-S-Ala or Ala-S-Abu which is formed by two "meLan" moieties present (either by replacement or insertion) in the stretch X4-X13. Thus, the annotation Lan pair or meLan pair comprised in X4-X13 refers to the final, cyclized structure. Biosynthetically, the Lan and meLan residues originate from cysteine, serine and threonine residues. First, selected serine and threonine residues are enzymatically dehydrated to form dehydroalanine (Dha) and dehydrobutyrine (Dhb) residues, respectively. Then, the nucleophilic addition of a cysteine thiol onto a Dha or Dhb residue in Michael manner forms a Lan or MeLan residue, respectively. These modifications establish a covalent linkage between a cysteine thiol and the β-carbon of another residue, thereby forming a thioether bridge. More specifically, the Ala-S-Ala structure can originate from Dha and Cys, wherein Cys can be N- or C-terminally located from Dha. The structure Abu-S-Ala or Ala-S-Abu from Dhb and Cys or Cys and Dhb, respectively.

US2013/0196899 provides synthetic cyclic analogs of apelin and methods fro providing them. The rings in the analogs of US2013/0196899 are formed by amino acids forming a disulfide or amide bond via their side chain or terminus, respectively. However, disulfides and amide bonds are chemically less stable than thioether bonds. Furthermore, during lanthionine formation stereochemistry plays a role, making the resulting peptide structure more defined and more receptor-specific. Also, thioether bridges within lanthionines are shorter bridges than disulfide bridges and than amide crosslinks. As a consequence, lanthionines induce structures that are different from those induced by disulfides and or amide crosslinks.

Hamada et al. (2008, Int. J. of Mol. Med. 22: 547-552) discloses the synthesis of cyclic analogs of the apelin fragment RPRLSHKGPMPF (apelin-12) wherein N-terminus is connected to the C-terminus, or wherein the sidechain of Lys at position 7 forms a ring with either the N- or C-terminal residue. In this case also cyclourea bonds occur, which are less stable than thioether bridges. Additionally, the variants of Hamada *et al*. have no free N- and/or no free C-terminus.

In a polypeptide analog each of X1 through X14 is to be chosen from a set of selected options.
X1 denotes the N-terminus of the polypeptide and is either absent or Lys-Phe-Arg-Arg. In one preferred aspect, X1 is absent.
X2 is either absent or selected from pGlu, Gln and Ala. Preferably, X1 is absent and X2 is Glu or pGlu (pyroglutamate). In a specific aspect, X1 is pGlu. Introduction of pyroglutamate at the N-terminus can protect the analog against N-terminal hydrolysis. Pyroglutamic acid (also known as PCA, 5-oxoproline, pidolic acid, or pyroglutamate for its basic form) is an uncommon amino acid derivative in which the free amino group of glutamic acid or glutamine cyclizes to form a lactam. It is a metabolite in the glutathione cycle that is converted to glutamate by 5-oxoprolinase. Pyroglutamate is found in many proteins including bacteriorhodopsin. N-terminal glutamic acid and glutamine residues can spontaneously cyclize to become pyroglutamate.
X3 is a basic amino acid residue selected from Arg and Ala, of which Arg is preferred.
X4 is selected from the group consisting of Pro, Ala, Pro-(me)Lan, Pro-Dhb, Ala-Dhb or Ala-(me)Lan; like Pro, Ala, Pro-(me)Lan or Ala-(me)Lan. In one embodiment, X4 is Pro or Ala, of which Pro is preferred. In another aspect, X4 is involved in thioether-bridge formation and selected from Pro-(me)Lan and Ala-(me)Lan. X4 can by Pro-Lan or Ala-Lan, such that the thioether bridge formed with a second Lan residue is a lanthionine of the structure Ala-S-Ala. Alternatively, X4 is Pro-meLan or Ala-meLan, such that the thioether bridge formed with a second meLan residue (e.g. (me)Lan, His-(me)Lan or Ala-(me)Lan at position X8) is a methyllanthionine of the structure Abu-S-Ala or Ala-S-Abu.
X5 is Arg, Ala, (me)Lan, Dhb, Arg-(me)Lan, Arg-Dhb, Ala-Dhb or Ala-(me)Lan; for example X5 is Arg, Ala, (me)Lan, Arg-(me)Lan or Ala-(me)Lan. Preferably, X5 is Arg or Ala, of which Arg is preferred. In another aspect, X5 is involved in thioether-bridge formation and selected from (me)Lan, Arg-(me)Lan and Ala-(me)Lan. X5 can be Lan, Arg-Lan or Ala-Lan, such that the thioether bridge formed with a second Lan residue (e.g. Lan, Lys-Lan or Ala-Lan at position X9) is a lanthionine of the structure Ala-S-Ala. Alternatively, X5 is meLan, Arg-meLan or Ala-meLan, such that the thioether bridge formed with a second meLan residue is a methyllanthionine of the structure Abu-S-Ala or Ala-S-Abu.
X6 is selected from the group consisting of Leu, Ala, (me)Lan, Dhb, Leu-(me)Lan, Leu-Dhb, Ala-Dhb and Ala-(me)Lan. For example X6 is Leu, Ala, (me)Lan, Leu-(me)Lan or Ala-(me)Lan. Preferably, X6 is Leu or Ala, of which Leu is preferred. In another aspect, X6 is involved in thioether-bridge formation and selected from (me)Lan, Leu-(me)Lan and Ala-(me)Lan. X6 can be Lan, Leu-Lan or Ala-Lan, such that the thioether bridge formed with a second Lan residue (e.g. Lan or Ala-Lan at position X10) is a lanthionine of the structure Ala-S-Ala. Alternatively, X6 is meLan, Leu-meLan or Ala-meLan, such that the thioether bridge formed with a second meLan residue is a methyllanthionine of the structure Abu-S-Ala or Ala-S-Abu.
X7 is selected from the group consisting of Ala, (me)Lan, Dhb, Ala-(me)Lan, Ala-Dhb, Dhb-Ala and (me)Lan-Ala. For example, X7 is Ala, (me)Lan, Ala-(me)Lan or (me)Lan-Ala. Preferably, X7 is involved in thioether-bridge formation and selected from (me)Lan, Ala-(me)Lan and Ala-(me)Lan. X7 can be Lan, Ala-Lan or Lan-Ala, such that the thioether bridge formed with a second Lan residue (e.g. Lan, Pro-Lan or Ala-Lan at position X11) is a lanthionine of the structure Ala-S-Ala. Alternatively, X7 is meLan, Ala-meLan or meLan-Ala, such that the thioether bridge formed with a second meLan residue is a methyllanthionine of the structure Abu-S-Ala or Ala-S-Abu. In a specific aspect, X7 is (me)Lan, optionally in combination with either X9 being Lys-(me)Lan, or X10 being (me)Lan or Ala-(me)Lan, X11 being (me)Lan or Ala-(me)Lan, X12 being (me)Lan, Met-(me)Lan or Ala-(me)Lan.
X8 is selected from the group consisting of His, Ala, (me)Lan, Dhb, His-(me)Lan, His-Dhb, Ala-Dhb and Ala-(me)Lan. For example, X8 is His, Ala, (me)Lan, His-(me)Lan or Ala-(me)Lan. Preferably, X8 is His or Ala, of which His is preferred. In another aspect, X8 is involved in thioether-bridge formation and selected from (me)Lan, His-(me)Lan and Ala-(me)Lan. X8 can be Lan, His-Lan or Ala-Lan, such that the thioether bridge formed with a second Lan residue (e.g. Lan, Met-Lan or Ala-Lan at position X12) is a lanthionine of the structure Ala-S-Ala. Alternatively, X8 is meLan, His-meLan or Ala-meLan, such that the thioether bridge formed with a second meLan residue is a methyllanthionine of the structure Abu-S-Ala or Ala-S-Abu. X8 consisting or comprising His is preferred.
X9 is selected from the group consisting of Lys, Ala, (me)Lan, Dhb, Lys-(me)Lan, Lys-Dhb, Ala-Dhb and Ala-(me)Lan. For example, X9 is Lys, Ala, (me)Lan, Lys-(me)Lan or Ala-(me)Lan. Preferably, X9 is Lys or Ala, of which Lys is preferred. In another aspect, X9 is involved in thioether-bridge formation and selected from (me)Lan, Lys-(me)Lan and Ala-(me)Lan. X9 can be Lan, Lys-Lan or Ala-Lan, such that the thioether bridge formed with a second Lan residue (e.g. Lan, Pro-Lan and Ala-Lan at position X13) is a lanthionine of the structure Ala-S-Ala. Alternatively, X9 is meLan, Lys-meLan or Ala-meLan, such that the thioether bridge formed with a second meLan residue is a methyllanthionine of the structure Abu-S-Ala or Ala-S-Abu. X9 consisting or comprising Lys is preferred. In a specific aspect, X9 is Lys-(me)Lan.
X10 is selected from the group consisting of Gly, Ala, (me)Lan, Dhb, Ala-Dhb and Ala-(me)Lan. For example, X10 is Gly, Ala, (me)Lan or Ala-(me)Lan. In one embodiment, X10 is Gly. In another embodiment, X10 is meLan.
X11 is Pro, Ala, (me)Lan, Pro-Lan or Ala-(me)Lan. Preferably, X11 is Pro or (me)Lan.
X12 is Met, Ala, (me)Lan, Met-(me)Lan or Ala-(me)Lan. Preferably, X12 is Met.
X13 is either absent or selected from Pro, Dhb, (me)Lan, Pro-(me)Lan and Ala-(me)Lan. Preferably, X13 is selected from Pro, Dhb, (me)Lan, Pro-(me)Lan and Ala-(me)Lan, more preferably from Pro and (me)Lan. In a specific aspect, X13 is Pro. In another specific aspect, X13 is Dhb.
X14 is the C-terminus and is either absent or is selected from Phe and Ala, unless X13 is absent. In one embodiment, X14 and X13 are absent. In another embodiment, X14 is Phe or Ala, of which Phe is preferred. In a specific aspect, the peptide variant is C-terminally amidated, for example wherein X14 is an amidated Phe (Phe-NH₂).

In a specific embodiment, X1 is absent and the sequence X2-X3-X4-X5-X6 is pGlu-Arg-Pro-Arg-Leu. Very good results were obtained with the sequence X7-X8-X9-X10 being (me)Lan-His-Lys-(me)Lan, preferably meLan-His-Lys-meLan, or with the sequence X7-X8-X9-X10 being (me)Lan-His-Lys-(me)Lan-Gly, preferably meLan-His-Lys-meLan-Gly.

In other variants, the sequence X4-X5-X6-X7-X8 is Pro-(me)Lan-Arg-Leu-Ala-(me)Lan or Pro-Arg-(me)Lan-Arg Leu-Ala-(me)Lan.

Exemplary variants include those wherein the four C-terminal residues consists of the sequence Pro-Met-Pro-Phe are preferred. However, in other useful variants X14 is absent or Ala, for example wherein the C-terminus is Pro-Met-Pro, Pro-Met-Pro-Ala. In yet another embodiment, X11-X12-X13-X14 is (me)Lan-Met-Pro-Phe.

Also encompassed are an amide, an ester or a salt of said polypeptide.

Preferably, the salt is a pharmaceutically acceptable salt, such as any nonpoisonous salt, for example, a salt with an inorganic acid, a salt with an organic acid, a salt with an inorganic base, a salt with an organic base and a salt with amino acid. As used herein, "acceptable salt" refers to salts that retain the desired activity of the oligopeptide or equivalent compound, but preferably do not detrimentally affect the activity of the oligopeptide or other component of a system in which uses the oligopeptide. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, trifluoroacetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminum, copper, cobalt, nickel and the like or with an organic cation formed from N,N'-dibenzylethylenediamine or ethylenediamine, or combinations thereof (e.g., a zinc tannate salt).

In one embodiment, a cyclic analog as provided herein has an APJ receptor potency similar to apelin-13 or pyr-1-apelin-13. In one embodiment, the cyclic APJ agonist of the present invention has an half-maximal effective concentration (EC₅₀) of less than 100 nM when measuring inhibition of cAMP production and / or stimulation of arresting recruitment and/or any other measurement of APJ-mediated signalling. In another embodiment, the EC₅₀ is less than 50 nM, preferably less than 25 nM and more preferably less than 15 nM. In a specific another embodiment, the analog of the present invention has an EC₅₀ of less than 10 nM.

The analog preferably has a plasma stability superior to apelin-13 or pyr-1-apelin-13. In one embodiment, the plasma stability improvement is at least 2-fold higher than what is observed for known apelins. Stability can be characterized by the half-life (t50), the time at which 50% of an initial enzymatic activity is preserved. In one embodiment, the analog of the invention has a half-life (t50) in plasma of at least 30 minutes. In another embodiment, the polypeptide of the invention has a half-life of at least 10 minutes, preferably at least 40 min and more preferably at least 60 minutes. In a specific embodiment, t50 in plasma is more than 1 hour, preferably more than 2 hours, most preferred at least 4 hours.

Specific apelin analogs of the present invention are shown in Table 1.

**Table 1**

| **Analog** | **Structure** |
|---|---|
| ApeI3c | QRP**meLan**RLA**meLan**KGPMPF |
| ApeI3cS | QRP**Lan**RLA**Lan**KGPMPF |
| ApeI4a | QRPR**meLan**LA**meLan**KGPMPF |
| ApeT4cS I*, | QRP**Lan**LAH**Lan**GPMPF |
| pEApeI3c | pERPmeLanRLAmeLanKGPMPF |
| pEApeI3cS | pERPLanRLALanKGPMPF |
| pEApeI4a | pERPRmeLanLAmeLanKGPMPF |
| pEApeT4cS I* | pERPLanLAHLanGPMPF |
| ApeM5 | QRPRL**meLan**HK**meLan** PMPF |
| pEApeM5 | pERPRL**melan**HK**melan**PMPF |
| pEApeM5T | pERPRL**meLan**HK**meLan**PMDhbF |
| ApeI5cS I* | QRPRL**Lan**AHK**Lan**PMPF |
| ApeI5cS II** | QRPRL**Lan**AHK**Lan**PMPF |
| ApeI5dS I* | QRPRL**Lan**AHK**Lan**GPMPF |
| ApeI5dS II** | QRPRL**Lan**AHK**Lan**GPMPF |
| pEApeI5cS I* | pERPRL**Lan**AHK**Lan**PMPF |
| pEApeI5cS II** | pERPRL**Lan**AHK**Lan**PMPF |
| pEApeI5dS I* | pERPRL**Lan**AHK**Lan**GPMPF |
| pEApeI5dS II** | pERPRL**Lan**AHK**Lan**GPMPF |
| ApeM6 | QRPRL**meLan**HK**meLan**GPMPF |
| pEApeM6 | pERPRL**meLan**HK**meLan**GPMPF |
| amidated pEApeM6 | pERPRLmeLanHKmeLanGPMPF-NH2 |
| ApeM6deltaF | QRPRL**meLan**HK**meLan**GPMP |
| pEApeM6deltaF | pERPRL**meLan**HK**meLan**GPMP |
| ApeM6S | QRPRL**Lan**HK**Lan**GPMPF |
| pEApeM6S | pERPRL**Lan**HK**Lan**GPMPF |
| pEApeM7 | pERPRL**meLan**HKG**meLan**PMPF |
| pEApeM7S I* | pERPRL**Lan**HK**GLan**PMPF |
| pEApeM7S II** | pERPRL**Lan**HK**GLan**PMPF |
| pEApeT8cS | pERPRLAH**Lan**GPM**Lan**F |
| ApeM7S I* | QRPRL**Lan**HKG**Lan**PMPF |
| ApeM7S II** | QRPRL**Lan**HKG**Lan**PMPF |
| ApeT8cS | QRPRLAH**Lan**GPM**Lan**F |
| ApeM8S | QRPRL**Lan**HKG**Lan**MPF |
| pEApeM8 | pERPRL**meLan**HKG**meLan**MPF |
| ApeM9S | QRPRL**Lan**HKGP**Lan**PF |
| ApeM12S | QRPRL**Lan**HKGP**Lan**MPF |
| pEApeM9S | pERPRL**Lan**HKGP**Lan**PF |
| pEApeM12S | pERPRL**Lan**HKGP**Lan**MPF |
| Ape17M2 | KFRRQRPRL**meLan**HK**meLan**PMPF |
| Ape17M3 | KFRRQRPRL**meLan**HK**meLan**GPMPF |
| Ape17M4 | KFRRQRPRL**Lan**AHK**Lan**PMPF |
| Ape17M5 | KFRRQRPRL**Lan**AHK**Lan**GPMPF |
| Ape17M6 | KFRRQRPRL**Lan**HKG**Lan**PMPF |
| Ape17M7 | KFRRQRPRLAH**Lan**GPM**Lan**F |
| ApeM26 | QRPRL**Lan**HKGPM**Lan**F |
| ApeM27 | QRPRL**Lan**HKGPM**Lan**PF |
| pEApeM26 | pERPRL**Lan**HKGPM**Lan**F |
| pEApeM27 | pERPRL**Lan**HKGPM**Lan**PF |

| | |
|---|---|
| * most frequent isomer resulting from spontaneous cyclization ** less frequent isomer resulting from spontaneous cyclization | |

Also provided is a method for providing an apelin analog according to the invention. Intramolecular linkages between the side chains of two amino acids in a cyclic peptide can be chemically or enzymatically formed. Cyclic apelin analogs are advantageously prepared using a host cell comprising one or more biosynthetic enzymes for lanthipeptide or lantibiotic synthesis (Arnison et al. 2013 Nat Prod Rep 30, 108-160). Lanthipeptides and lantibiotics (a subgroup of lanthipeptides with antimicrobial activity) are small peptides containing internal bridges resulting from the formation of (methyl)lanthinione or lysinoalanyl residues (reviewed in McAuliffe et al., FEMS Microbiol. Rev. 25,285-308 (2001). Lanthionine (Lan) and methyllanthionine (MeLan) result from the dehydration of serine to dehydroalanine (Dha) and of threonine to dehydrobutyrine (Dhb), respectively, and thioether bond formation resulting from the interaction of these amino acids with a cysteine residue within the same peptide. Well known examples of lantibiotics are nisin, subtilin, Pep5, and epiderminin. Lantibiotics are ribosomally synthesized as inactive prepeptides, containing an amino terminal leader peptide and a carboxy terminal propeptide. The leader peptide is necessary for the interaction of the lantibiotic precursor with lantibiotic enzymes. The leader peptide is proteolytically cleaved from the propeptide either inside the producing cell, or during or after transport out of the cell. The lanthipeptide biosynthetic genes are clustered together and are given the locus name lan. *LanA* is the gene encoding the lantibiotic prepeptide, *lanB* is the gene encoding an enzyme responsible for serine or threonine dehydration and *lanC* is the gene encoding an enzyme responsible for cycle formation. In some cases, the activities of LanB and LanC are joined in a bifunctional enzyme encoded by *lanM. lanP*, and *lanT*, genes encode enzymes that are involved in processing and/or transport of prepeptides.

Accordingly, the invention provides a method for the enzymatic preparation of a cyclic apelin analog according to the invention, comprising:
providing a host cell comprising:
   - a nucleic acid molecule comprising a first nucleic acid fragment encoding an N-terminal leader peptide found with the precursor peptide of a lanthipeptide or lantibiotic and a second nucleic acid fragment encoding an apelin analog, whereby said first and second fragment are within the same open reading frame of said nucleic acid molecule;
   - a nucleic acid sequence encoding an enzyme capable of dehydrating serine and/or threonine;
   - optionally a nucleic acid sequence encoding a transporter protein; and
   - a nucleic acid sequence encoding an enzyme capable of inducing lanthionine or methyllanthionine formation.

Any leader peptide found with the precursor of a lanthipeptide/lantibiotic is suitable for use in a method for the preparation of cyclic peptide analogs according to the invention. Examples of such leader peptides are the leader peptides of nisin, subtilin, Pep5, and epiderminin. In a preferred embodiment, a nisin leader peptide is used. The enzyme capable of dehydrating serine and/or threonine preferably is a LanB, such as NisB, PepB, SpaB, or a functional equivalent thereof. The enzyme capable of inducing lanthionine and/or methyllanthionine formation preferably is a LanC, such as NisC, PepC, SpaC, or a functional equivalent thereof. In one embodiment, dehydration of serine and/or threonine and lanthionine and/or methyllanthionine formation are induced by the same enzyme, a LanM, such as, CinM, LtnM, LctM, or a functional equivalent thereof.

Lanthionine formation between dehydroalanine and cysteine is energetically possible at room temperature and can also occur spontaneously, without the involvement of a LanC or LanM enzyme.

Therefore, in one embodiment, a method is provided for the enzymatic preparation of a cyclic apelin analog according to the invention comprising a (methyl)lanthionine bridge comprising:
a) providing a host cell comprising:
   - a nucleic acid molecule comprising a first nucleic acid fragment encoding an N-terminal leader peptide found within the precursor peptide of a lanthipeptide/lantibiotic and a second nucleic acid fragment encoding an apelin analog, whereby said first and second fragment are within the same open reading frame of said nucleic acid molecule;
   - a nucleic acid sequence encoding an enzyme capable of dehydrating serine and/or threonine;
   - (optionally) a nucleic acid sequence encoding a transporter protein; and
b) allowing for the translation of said first nucleic acid;
c) harvesting said peptide analog, preferably harvesting the peptide either from the medium or after host cell lysis from the cytoplasm.

Thus, a (methyl)lanthionine moiety can be installed post-translationally into a linear precursor peptide via enzymatic dehydration of serine and threonine to the α,β-unsaturated residues 2,3-didehydroalanine (Dha) and (Z)-2,3-didehydrobutyrine (Dhb), respectively, followed by intramolecular Michael-type addition of cysteine thiol to yield the thioether cross-link. This approach using the biosynthetic machinery can be reconstituted *in vitro* or in bacterial hosts.

In one embodiment, a method comprises establishing a host cell with a plasmid coding for the lanthipeptide modification enzymes that dehydrate serines/threonines and couple the formed dehydroamino acids to cysteine and optionally coding for a lanthipeptide transporter and with a plasmid coding for a construct composed of an N-terminal lanthipeptide leaderpeptide and a C-terminal apelin analog.

As detailed above, a (methyl)lanthionine is formed between the side chains of a dehydrated serine or threonine, i.e. a dehydroalanine or a dehydrobutyrine respectively, and a cysteine. Therefore, a nucleic acid sequence encoding an apelin analog which (when cyclized) comprises at least one lanthionine or methyllanthionine, needs to have a nucleic acid codon encoding a serine (TCT, TCC, TCA, TCG, AGT or AGC) or a threonine (ACT, ACC, ACA or ACG) and a nucleic acid codon encoding a cysteine (TGT or TGC) at the two amino acid positions within the peptide analog which will form the lanthionine or methyllanthionine.

In one embodiment, the second nucleic acid fragment encoding an apelin analog encodes a polypeptide selected from the group consisting of
QRPTRLACKGPMPF, QRPSRLACKGPMPF, QRPRTLACKGPMPF, QRPSLAHCGPMPF, QRPRLTHKCPMPF, QRPRLSAHKCPMPF, QRPRLSAHKCPMPF, QRPRLSAHKCGPMPF, QRPRLSAHKCGPMPF, QRPRLTHKCGPMPF, QRPRLTHKCGPMPF-NH₂, QRPRLTHKCGPMP, QRPRLSHKCGPMPF, QRPRLTHKGCPMPF, QRPRLSHKGCPMPF, QRPRLSHKGCPMPF, QRPRLAHSGPMCF, QRPRLSHKGCMPF, QRPRLTHKGCMPF, QRPRLSHKGPCPF, QRPRLSHKGPCMPF, KFRRQRPRLTHKCPMPF, KFRRQRPRLTHKCGPMPF, KFRRQRPRLSAHKCPMPF, KFRRQRPRLSAHKCGPMPF, KFRRQRPRLSHKGCPMPF, KFRRQRPRLAHSGPMCF, QRPRLSHKGPMCF and QRPRLSHKGPMCPF.

In case the N-terminal residue of the peptide is glutamine (Q), the method preferably further comprises conversion of Q to pE according to methods known in the art, for example as described in Rink et al. (Journal of Pharmacological and Toxicological Methods 61 (2010) 210-218).

For the purpose of harvesting a cyclic peptide analog or a dehydrated peptide analog that has not yet been cyclized from the medium, it is especially preferred that a transporter protein is present if the host cell is a Gram-positive bacterium. Preferably said transporter protein is a LanT, such as NisT, or a functional equivalent thereof. In *Escherichia coli* the presence of a transporter is not required and harvesting of the peptide analog preferably follows disruption of the cells.

A host cell used in a method for the preparation of a cyclic analog according to the invention is preferably a Gram-positive prokaryote, a Gram-negative prokaryote or an eukaryote. Examples of suitable host cells include lactic acid bacteria, such as *Lactococcus lactis, Bacillus subtilis*, *Staphylococcus epidermis* and the Gram-negative bacterium *E*. *coli.*

Instead of enzymatic cyclization coupling of a dehydroamino acid with a cysteine, cyclization is also possible by incubation at alkaline pH. This pH - induced coupling is advantageously used to form (methyl)lanthionines for instance when a proline is directly preceding a cysteine which precludes efficient cyclase activity. Alternatively, in the case of peptides which are not cyclized by the cyclase NisC, cyclization between dehydroalanine and cysteine can take place spontaneously which -depending on the peptide- may or may not result in different isomers. Hence, in an alternative embodiment of the invention, ring closure is achieved in a non-enzymatic fashion, for example by exposure of a dehydrated precursor analog comprising a dehydroalanine or a dehydrobutyrinee residu and a cysteine residue at the desired positions to a chemical treatment inducing the formation of a thioether bridge. For example, chemical ring closure may involve exposure of a peptide comprising a dehydroalanine and a cysteine during at least 10 minutes to pH 8 (Burrage et al Chem Eur J, 2000 Biomimetic synthesis of lantibiotics. 6, 1455- 1466) or of a peptide with a dehydrobutyrine and a cysteine during about 10 hours to pH 8-9 (Zhu et al 2003 Biomimetic studies on the stereoselective lanthionine formation. Org. Biomol. Chem. 1, 3304-3315).

In yet another embodiment, the apelin analog is synthesized by solid-supported chemical synthesis, such as according to the procedure described by Knerr et al. (J. Am. Chem. Soc., 2012, 134 (18), pp 7648-7651) or references cited therein.

In view of its (differential) effect on the APJ receptor combined with increased stability, a cyclic apelin analog as provided herein advantageously finds its use as medicament. Therefore, also provided is a pharmaceutical composition comprising at least one apelin analog according the invention and a pharmaceutically acceptable carrier, diluent or excipient. The analog can be administered as the entity, as such, or as a pharmaceutically acceptable acid- or base addition salt, formed by reaction with an inorganic acid (such as hydrochloric acid, hydrobromic acid, perchloric acid, nitric acid, thiocyanic acid, sulfuric acid, and phosphoric acid); or with an organic acid (such as formic acid, acetic acid, propionic acid, glycolic acid, lactic acid, pyruvic acid, oxalic acid, malonic acid, succinic acid, maleic acid, and fumaric acid); or by reaction with an inorganic base (such as sodium hydroxide, ammonium hydroxide, potassium hydroxide); or with an organic base (such as mono-, di-, trialkyl and aryl amines and substituted ethanolamines). As a cyclic peptide compound or a pharmaceutically acceptable salt thereof in the present invention, a compound or a pharmaceutically acceptable salt thereof in a substantially purified form is preferred. Over 80% or more of purities is more preferable.

The apelin receptor APJ is associated with cardiovascular disorders, atherosclerosis, restenosis, ischemic cardiovascular disease, viral cardiomyopathy, lymphatic vessel stabilization, endocrine system and hormonal disorders, diabetic retinopathy and metabolic diseases, gastrointestinal and liver diseases, cancer disorders, inflammatory diseases, haematological disorders, respiratory diseases, muscle-skeleton disorders, neurological disorders, urological disorders, dermatological diseases, wound healing and reproduction disorders.

The invention provides (methyl)lanthionine-containing agonists and antagonists of the APJ receptor, whose selective application depends on the disease. It is furthermore envisaged that lanthionine apelins affect known heterodimerization of the APJ receptor with the AT1 receptor with therapeutic relevance.

For apelin receptor agonists multiple therapeutic areas exist. Primary targets for the neovascularization properties of apelin are heart and leg muscle. The APJ receptor stimulation generally has cardiovascular therapeutic relevance. Other fields of applications are for instance fibrosis, to which (methyl)lanthionine-apelin may have reverting properties, and neuroprotection. Apelin has therapeutic effects in neonatals exposed to hyperoxia. In other diseases for instance cases of pathological neoangiogenesis apelin receptor antagonists are valuable: diabetic nephropathy (Zhang 2013 PLOS 8 | Issue 4 | e60457 page 1-11); antitumor growth (Sorli 2006 Drug discovery today 11, 1100-1106); Ischemic retinopathy. Antagonists are obtained for instance by replacing F13 with ala13. Blocking the APJ receptor, which is co-receptor for HIV infection, contributes to prevention of HIV infection. Blocking the APJ receptor may contribute to reducing the pathological formation of excedentary vessels observed in age-related macular degeneration (AMD).
Thus, exemplary therapeutic applications include all those known or suggested for being associated with signaling via the apelinergic system signaling, for instance conditions related to altered water balance, stress-induced disorders such as anxiety and depression, cardiovascular and metabolic disorders. In one embodiment, the apelin analog produces endothelium-dependent vasodilation, endothelium-independent vasoconstriction and/or an increase force of heart contraction. Apelin has anti-inflammatory effects in hyperoxia treated rat pups. The invention provides a cyclic apelin analog for use in a method for the treatment or prevention of a cardiovascular condition, for instance selected from the group consisting of hypoxia, ischaemia and pulmonary arterial hypertension. In a further embodiment, the apelin analog finds its use in modulation of angiogenesis, e.g. in tumors.

APJ is also a human immunodeficiency virus type I (HIV-1) co-receptor, and apelin blocks HIV-1 entry through APJ. The inhibition of HIV infection in CHO cells and NP-2 cells expressing CD4 and receptor after preincubation with apelin peptides supports a role for APJ as a co-receptor for HIV and HIV-2. Incubation of cells negative for CD4 but positive for APJ with soluble CD4 enabled HIV infection. Accordingly, in still a further embodiment, a cyclic apelin of the invention is used for the treatment of HIV.

As used herein, "treatment" means alleviating or healing symptoms or disease, and/or its accompanying symptoms. "Prevention" means a method of delaying or preventing the onset of symptoms or disease, and/or its accompanying symptoms, a method of keeping a patient from acquiring symptoms or disease, or a method of reducing a risk of a patient acquiring symptoms or disease.

The pharmaceutical composition of the present invention is manufactured by suitably mixing a compound or a pharmaceutically acceptable salts thereof in the present invention with at least one or more sorts of pharmaceutically acceptable carriers etc. in suitable amounts in accordance with known methods in the technical field of medicinal preparation. Content amounts of a compound or a pharmaceutically acceptable salt thereof in the present invention in the pharmaceutical composition change depending on dosage forms, dose, etc., but are 0.1 to 100% of the weight to the whole pharmaceutical composition, for example.

A "pharmaceutical composition" include oral preparations such as tablets, capsules, granules, powders, trochiscus, syrups, emulsion, and suspension, and parenteral preparations such as external preparations, suppositories, injections, ophthalmic solutions, intranasal agents, and pulmonary agents.
"Pharmaceutically acceptable carriers" includes various conventional organic or inorganic carrier substances, for example, substances in solid preparations such as excipients, disintegrators, binders, glidants and lubricants, and substances in liquid preparations such as solvents, solubilizing agents, suspending agents, isotonizing agents, buffers and soothing agents. Additives such as preservatives, antioxidants, colorants, and edulcorants, are used if needed. The "excipients" includes, for example, lactose, white soft sugar, D-mannitol, D-sorbitol, corn starch, dextrin, microcrystalline cellulose, crystalline cellulose, carmellose, carmellose calcium, carboxy-methyl-starch sodium, low substituted hydroxypropylcellulose and acacia. Glidants include, for example, light anhydrous silicic acid and magnesium stearate. Lubricants include, for example, magnesium stearate, calcium stearate and talc. The solvents include, for example, purified water, ethanol, propylene glycol, macrogol, sesame oil, corn oil and olive oil. Solubilizing agents include, for example, propylene glycol, D-mannitol, benzyl benzoate, ethanol, triethanolamine, sodium carbonate and sodium citrate. The "suspending agents" include, for example, benzalkonium chloride, carmellose, hydroxypropylcellulose, propylene glycol, povidone, methylcellulose and glyceryl monostearate. The buffers include, for example, disodium hydrogen phosphate, sodium acetate, sodium carbonate and sodium citrate.

The pharmaceutical composition of the present invention can be administrated to mammals other than human (for example, mice, rats, hamsters, guinea pigs, rabbits, cats, dogs, pig, cows, horses, sheep, monkeys) and to human, in oral or parenteral (for example, topical, rectum, intravenous administration) in a therapeutically effective amount. Although the "therapeutically effective amount" changes depending on patients, disease, symptoms, dosage forms, routes of administration, for example, the dose in the case of administering orally to the adult patient (weight: about 60 kg) suffering from a cardiovascular disease ranges usually from about 1 mg to 1 g per day using, as an active ingredient, a cyclic apelin analog or a pharmaceutically acceptable salt thereof in the present invention. Such quantity can be administrated to the patient once or in several times a day.

The pharmaceutical composition comprising a compound or a pharmaceutically acceptable salt thereof in the present invention as an active ingredient or an activator, and the kits (administration, treatment and/or prevention kit), packages (packaging goods etc.), and medicine set (and/or, container) containing the package insert about the pharmaceutical composition which indicate that the pharmaceutical composition can be used or should be used for treatment and/or prevention are also useful. Such kit, the package, and the medicine set may be provided with one or more containers filled with one or more active ingredients and other medicines, or the medicine (or the component) for the above-mentioned pharmaceutical compositions. As examples of such a kit, a package, and a medicine set, the kit for commerce appropriately directed to the treatment and/or prevention of an object disease and the package for commerce are included. As a package insert comprised in such a kit, a package, and a medicine set, notes by the government organization which regulates manufacture, use or sale of a pharmaceutical or biological products, and notes which show the approval of the government organization about manufacture, use or sale of the product relevant to medication to a human are included. In the above-mentioned kit, package and medicine set, the packed product may also be included, and the structure constituted by adopting a suitable medication step (step) may be included, and the structure constituted as could attain the treatment and/or prevention on more preferable medicine including treatment, prevention of an object disease may be included.

A compound or a pharmaceutically acceptable salt thereof in the present invention may be used by the general method currently performed in the medicinal field in combination (henceforth "combination therapy") with one or multiple other drugs (henceforth, a "concomitant drug"). A timing for administration of an apelin analog or a pharmaceutically acceptable salt thereof in the present invention and a concomitant drug is not limited. They may be administrated to the patient as a combination drug, or they may be administrated to the patient simultaneously or in a constant interval. A pharmaceutical kit which is characterized in consisting of a pharmaceutical composition of the present invention and a concomitant drug can be used. The dose of a concomitant drug should comply with the dose in clinical use, and it can be selected suitably depending on patients, disease, symptoms, dosage forms, routes of administration, administration time, combination. The administration method of a concomitant drug is not limited in particular, and a compound or a salt thereof in the present invention and a concomitant drug should just be put together.

The concomitant drug includes, for example,
(1) a therapeutic agent and/or prophylactic of a cardiovascular disorder, atherosclerosis, restenosis or ischemic cardiovascular disease
(2) a therapeutic agent and/or prophylactic of endocrine system and hormonal disorders, diabetic retinopathy or metabolic disease
(3) a therapeutic agent of cancer,
(4) a therapeutic agent and/or prophylactic of an inflammatory disease, and
(5) a therapeutic agent of HIV, and any one or multiple of these agents and a compound or a pharmaceutically acceptable salt thereof in the present invention may be used in combination.

For the purpose of clarity and a concise description features are described herein as part of the same or separate embodiments, however, it will be appreciated that the scope of the invention may include embodiments having combinations of all or some of the features described. In view of this passage it is evident to the skilled reader that the variants of claim 1 as filed may be combined with other features described in the application as filed, in particular with features disclosed in the dependent claims, such claims usually relating to the most preferred embodiments of an invention.

### EXPERIMENTAL SECTION

### EXAMPLE 1: Synthesis of lanthionine-stabilized apelin analogs

Lanthionine containing apelin variants were made according to established procedures described for example in Kluskens, L.D., et al (2005) Post-translational Modification of Therapeutic Peptides by NisB, the Dehydratase of the Lantibiotic Nisin. Biochemistry 44, 12827-12834; Kluskens, L.D., et al. (2009) Angiotensin-(1-7) with thioether-bridge: an ACE-resistant, potent Ang-(1-7) analogue. J. Pharmacol. Exper. Ther. 328, 849-854; Rink, R. et al. (2007) NisC, the cyclase of the lantibiotic nisin, can catalyze cyclization of designed non-lantibiotic peptides. Biochemistry 46, 13179-13189.

Briefly, *Lactococcus lactis* comprising a two plasmid system was used. The first plasmid encoded the leader peptide of the lantibiotic nisin
MSTKDFNLDLVSVSKKDSGASPR genetically fused at its C-terminus to the precursor of the aimed for (methyl)lanthionine-apelin which contains at position i a serine/threonine and at position i+3, i+4, i+5 or i+6 a cysteine. Alternatively, the first plasmid coded for nisin leader followed by the first 17 amino acids of nisin and a endoproteinase Glu-C cleavage site (E):
MSTKDFNLDLVSVSKKDSGASPRITSISLCTPGCKTGALM IE. The N-terminal methionine is usually cleaved off by the host.

The plasmid encoding the fusion peptide comprising the nisin leader and the (methyl)lanthionine apelin precursor was co-expressed in *L. lactis* with the second plasmid pIL3BTC plasmid, encoding the maturation enzymes NisB and NisC, and the translocation enzyme NisT. NisB dehydrated the serine or threonine to yield dehydroalanine and dehydrobutyrine, respectively. Subsequently, the cyclase NisC covalently coupled the dehydroamino acid to cysteine, yielding a lanthionine or methyllanthionine respectively. The *L. lactis* culture was grown overnight in GM17 medium supplemented with 4 µg/ml Erythromycin and 4 µg/ml Chloroamphenicol at 30 °C (Kluskens LD, et al. (2005). Post-translational modification of therapeutic peptides by NisB, the dehydratase of the lantibiotic nisin. Biochemistry 44:12827-12834). 1/100 volume was then passed to Minimal Medium and culture was grown overnight at 30 °C (Rink R, et al. (2005) Lantibiotic structures as guidelines for the design of peptides that can be modified by lantibiotic enzymes. Biochemistry 44:8873-8882)

The supernatant was collected by centrifugation (10 min, 12857g), and was subsequently vacuum-filtered (0.2 µm). The peptide was purified from the medium fraction using HiTrap SP HP ion-exchange chromatography columns (GE Healthcare, Sweden). The collected fraction including the peptide was desalted (PD10 column, Amersham) and vacuum-dried.

The (methyl)lanthionine-apelin peptide was released by proteolytic digestion with 10 U / ml Endoproteinase Glu-C in 50 mM sodium phosphate buffer pH 7.6 at 37 °C. Incubation for 48 hours at said temperature results in the full conversion of an N-terminal glutamine (Q) into pyroglutamate (pE). If a N-terminal Q is required, the digestion time by Glu-C is kept to a minimum of a few hours. Digested fusion peptide was separated on a reversed-phase high-performance liquid chromatography (HPLC) column (Jupiter 4 µm Proteo 90Å, C12, Phenomenex). Separation was carried out at 1.0 mL/min in a gradient from 15 to40% acetonitril in milliQ water, in the presence of 0.1% trifluoroacetic acid. Peak fractions (detected at 214 nm) were collected and analyzed by Maldi-TOF mass spectrometry (Voyager-DE Pro, Applied Biosystems).
The absence or presence of a (methyl)lanthionine was assessed by incubation with CDAP, which adds to unmodified cysteines but not to (methyl)lanthionines (Rink 2007 Biochemistry 46, 13179-13189).

In some cases, dehydrated serine was coupled to cysteine in a non-enzymatic manner, resulting in different isomers. Lanthionines (Ala-S-Ala) are indicated as [Lan-Lan]; methyllanthionines (Abu-S-Ala and Ala-S-Abu) as [meLan-meLan].

### EXAMPLE 2: Binding of lanthionine-apelin variants to the APJ receptor

### Methods: APJ competition binding assay

[Glp⁶⁵, Nle⁷⁵, Tyr⁷⁷][¹²⁵I]-Apelin 13 binding experiments are carried out in SPA 96-well format. Membranes used in this assay are prepared from HEK293 cells stably expressing either recombinant hAPJ or rAPJ. The incubation is initiated by the addition of a mixture of WGA PVT SPA beads (0.5 mg/well) and 0.08 µg of membranes to an assay buffer (25 mM HEPES, 10 mM MgCl₂, ImM CaCl₂, 0.1% BSA, pH7.4) containing 0.06 nM [Glp⁶⁵, Nle⁷⁵, Tyr⁷⁷][¹²⁵I]-Apelin 13 and increasing concentrations of the tested compound (10 point concentration response curves). Non-specific binding is determined in the presence of 100 nM apelin 17. Samples are incubated during eight hours at room temperature (22°C). Then, 96-well plates are read in a Microbeta Trilux.

### Results:

**Table 2**

| hAPJ BINDING | |
|---|---|
| peptide | Relative IC₅₀ |
| QRPRLSHKGPMPF (wt, pos control) | 0.25 |
| | |
| SGASPR::nisA-(1-17)::IE (neg control) | >1000.0000 |
| pERPRLmeLanHKmeLanGPMPA (F to A mutation) | >1000.0000 |
| | |
| pERPRLmeLanHKGmeLanMPF | >1000.0000 |
| pERPRLmeLanHKmeLanGPMVF (P to V mutation) | >1000.0000 |
| | |
| pEApeM5 | 115.34 |
| pEApeM6 | 185.86 |

| | |
|---|---|
| pE is pyroglutamate. | |

### Conclusions

- P to A or P to V mutations abolish binding. However, Example 8 herein below shows significant activity of QRPRLAHLanGPMLanF. Hence, the helix breaking effect of a proline can be mimicked by a lanthionine-bridge. F to A mutant does not bind.
- Two lanthionine-containing apelins have retained the capacity to bind to the APJ receptor. Their binding capacity is lower than that of wild type apelin13. It should be noted however that the lanthionine apelins have a pE instead of a Q, which may partly account for reduced receptor binding.

### EXAMPLE 3 : Lanthionine apelins affect cAMP levels in cells expressing the APJ receptor.

### Methods: Forskolin-stimulated cAMP assay

Cyclic AMP level in HEK293 cells expressing recombinant APJ receptors is assayed by an HTRF competitive immunoassay. Cells at a density of 4000cells/well are resuspended in assay buffer (HBSS containing Ca²⁺ and Mg²⁺ supplemented with 0.1%BSA and 5mM HEPES) and added to the assay plate. Incubation for 40 min at room temperature is started with the addition of increasing concentrations of the compounds containing 10µM forskolin and 0.5mM IBMX. Reaction is stopped by addition of lysis buffer including d2-labeled cAMP, followed by addition of cryptate-labeled anti-cAMP monoclonal antibody, according to the manufacturer instructions. Time-resolved fluorescence at 620 and 665 nm is measured in an Envision instrument after 2 hour incubation at room temperature in the dark. cAMP content in the samples is determined by interpolation in a cAMP standard curve.

### Results:

**Table 3**

| cAMP | |
|---|---|
| peptide | Relative EC₅₀ (nM) |
| QRPRLSHKGPMPF | 0.017 |
| | |
| SGASPR::nisA-(1-17)::IE (neg control) | >300.0000 |
| pERPRLmeLanHKmeLanGPMPA | >300.0000 |
| | |
| pERPRLmeLanHKGmeLanMPF | >300.0000 |
| pERPRLmeLanHKmeLanGPMVF | >300.0000 |
| | |
| pEApeM5 | 29.265 |
| pEApeM6 | 19.585 |

### Conclusion:

The two variants which bind the receptor as demonstrated in example 1, are active with respect to modulation of cAMP level.

### EXAMPLE 4: Lanthionine apelins affect arrestin recruitment

### Methods:

### hAPJ β-arrestin recruitment assay

Stimulation of hAPJ interaction with β-arrestin is studied using the PathHunter™ system from DiscoveRx. CHO-K1 cell line was engineered to co-express the ProLink/Enzyme donor (PK)- tagged APJ and the enzyme activator (EA)-tagged β-arrestin fusion proteins. Upon GPCR activation, enzyme fragment complementation occurs, to produce an active β-Gal enzyme. These cells are seeded at a density of 15000 cells/well. Next day, cells are challenged with compounds with potential agonist profile at different concentrations. Following 90 min incubation at 37°C with the compounds, cells are lysed and incubated with PathHunter reagent for 1 hour at room temperature. Samples are then read with an ultrasensitive luminescence protocol using an Envision instrument (Perkin Elmer).

### Results:

**Table 4**

| Arrestin recruitment | |
|---|---|
| peptide | Relative EC₅₀ |
| QRPRLSHKGPMPF (wt, pos control) | 0.4895 |
| | |
| SGASPR::nisA-(1-17)::IE (neg control) | >300.0000 |
| pERPRLmeLanHKmeLanGPMPA (neg control) | >300.0000 |
| | |
| pERPRLmeLanHKGmeLanMPF | >300.0000 |
| pERPRLmeLanHKmeLanGPMVF | >300.0000 |
| | |
| pEApeM5 | 14.0877 |

### Conclusions:

The pEApeM5 variant has significant activity in this assay.

### EXAMPLE 5 : Lanthionine-containing apelins do not interact with the

### angiotensinII type 1 (AT₁) receptor.

In view of the homology between the APJ and the AT1 receptor it was investigated whether the lanthionine-apelins interact with the angiotensinII type 1 receptor. For example, it would be undesirable for various therapeutic applications if the lanthionine variants of apelin stimulated the AT1 receptor.

### Methods:

Agonistic and antagonistic action of lanthionine apelins was measured via AT1 coupled beta-arrestin assays.

### Results:

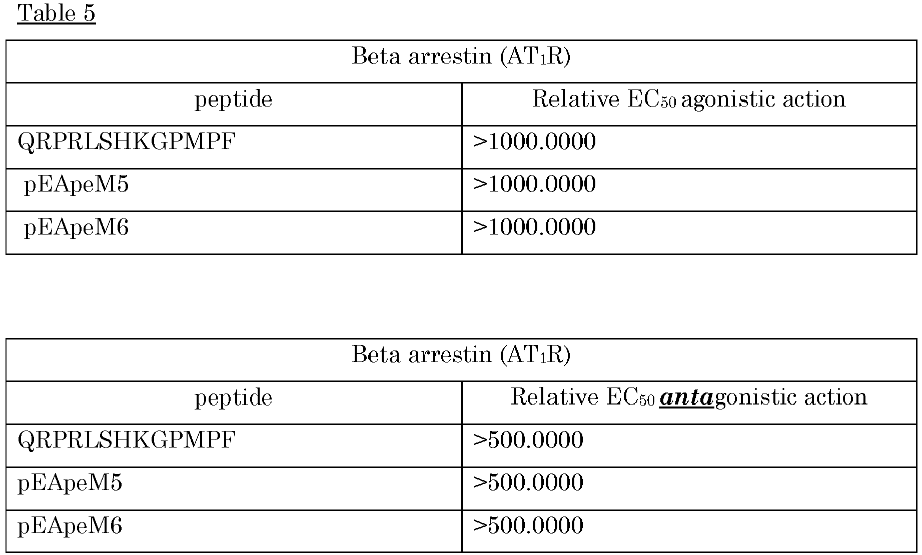

### Conclusion:

The lanthionine apelins did neither agonistically nor antagonistically act on the AT1 receptor.

### EXAMPLE 6: hAPJ receptor internalization assay

### Methods:

HEK293 cells are seeded 24 hours prior to experiment at 50% confluence in 2 x F75 flasks. The next morning, cells are trypsinized, vortexed for 60 seconds and passed through a 40micron mesh filter. Cells are resuspended at a density of 300,000 cells/ml in OptiMEM + 2% FBS, and Ad-GFP-hAPJ virus is added at an MOI of 1 PFU/cell. Cells are vortexed and dispensed into 96 well clear bottom, black dishes at a concentration of 10,000 cells/well. Cells are maintained at room temperature for 30 minutes to allow cell attachment to the dish prior to Incubation overnight at 37°C in a 5% CO₂ incubator. Next morning, apelin analogs are added in a concentration response curve (500 nM max). APJ-GFP translocation is observed and documented on Olympus FV con-focal microscope. A typical translocation run is completed in 3 hours with most of the effects observed in the initial 90 minutes of compound exposure.

### Results:

**Table 6**

| hAPJ internalisation | |
|---|---|
| peptide | Relative EC₅₀ (nM) |
| pERPRLSHKGPMPF | 0.22 |
| QRPRLSHKGPMPF | 0.58 |
| | |
| pERPRLmeLanHKGmeLanMPF | >1000 |
| pERPRLmeLanHKmeLanGPMVF | >1000 |
| pERPRLmeLanHKmeLanGPMP | >1000 |
| | |
| pEApeM6 | 207 |
| pERPRLmeLanHKmeLanGPMPA | 36 |
| pEApeM7 | 33 |
| | |
| pEApeM5 | 1.25 |

### Conclusion:

Since internalization may reflect desensitization, variants which show signalling capacity but have reduced internalization are preferred. In this series, taking into account the cAMP signalling in example 2, pEApeM6 is preferred.

### EXAMPLE 7: Lanthionine apelin has increased resistance to breakdown by peptidases in plasma.

### Methods:

Stability of apelin analogs in rat plasma. Apelin (0.1 mg /ml apelin final concentration) was incubated in 10% Rat plasma which was buffered with 16 mM phosphate buffer of 7.4 at 37 °C. Samples were quenched by acidifying to final concentration of 5% TFA. Samples were analyzed on HPLC. Peak heights were plotted as relative to the initial peak heights.

### Results:

**Table 7**

| **peptide** | **T50 stability in rat plasma (hr)** |
|---|---|
| QRPRLSHKGPMPF | 1 |
| pERPRLSHKGPMPF | 3 |

| **Cyclic apelins** | |
|---|---|
| pEApeI3c | 2 |
| pEApeI4a | 2 |
| pEApeI5dS I | 2 |
| pEApeI5cS I | 3 |
| pEApeM12S | 3 |
| pEApeM6deltaF | 3 |
| pEApeM7S I | 6 |
| pERPRL[AbuHKA]GPMPA | 7 |
| pEApeT8cS | 8 |
| pEApeM6 | 8 |
| pEApeM5 | 9 |
| pEApeM6deltaF | 10 |
| pERPRL[AbuHKA]GPMVF | 10 |
| pEApeM7 | 15 |
| pEApeM8 | 24 |
| pEApeM5T | 24 |
| pEApeM26 I | > 24 |
| pEApeM26 II | > 24 |
| pEApeM27 I | > 24 |
| pEApeM27 II | > 24 |

### Conclusion:

Clearly peptide with pGlu and lanthionine is much more stable than the linear peptide with Q. Both the pGlu and the lanthionine contribute to stability.

Comparison of variant pEApeM5T with pEApeM5 demonstrates enhanced stability by the Dhb residue.

### EXAMPLE 8: Inhibition of the cAMP pathway and stimulation of the B-arrestin pathway by stimulating the APJ receptor.

(Me)lan apelin variants were tested with cAMP and beta arrestin assays from the company DiscoverX.

**Table 8**

| | cAMP EC₅₀ (nM) | β-arrestin EC₅₀ (nM) | Ratio β-arrestin/ cAMP |
|---|---|---|---|
| QRPRLSHKGPMPF | 0.2 - 0.5 | 2.2 - 2.8 | 7 |
| QRPRLAHKGPMPF (control) | 0.6 | 12 | 20 |
| | | | |
| pEApeT4cS I | 33 | 740 | 22 |
| pEApeI3c | 17 | 2840 | 167 |
| pEApeI4a | 12.8 | 1110 | 87 |
| pEApeM5 | 7.0 | 67 | 9 |
| pEApeM6 | 21 | 93 | 4 |
| pEApeM7 | 27 | 500 | 18 |
| pEApeM7S I | 1.3 | 62 | 47 |
| pEApeM7S II | 2.6 | 255 | 98 |
| pEApeI5cS I | 2.5 | 149 | 59 |
| pEApeI5cS II | 2.3 | 34 | 14 |
| pEApeI5dS I | 2.1 | 616 | 293 |
| | 2.4 | 155 | 64 |
| pEApeI5dS II | | | |
| pEApeT8cS | 2.0 | 245 | 122 |
| pEApeM5T | 190 | 32 | 0.16 |
| pEApeM6deltaF | 125 | 385 | 3 |
| amidated pEApeM6 | 80 | 60 | 0.75 |
| pEApeM9S I | 18 | 520 | 84 |
| pEApeM9S II | 0.2 | 40 | 200 |
| pEApeM12S | 600 | >10000 | >16 |
| pEApeM8 | 125 | >1000 | >8 |
| pEApeM26S I | 0.6 | 5.3 | 8.8 |
| pEApeM26S II | 1.0 | 4.2 | 4.2 |
| pEApeM27S I | 3 | 149 | 50 |
| pEApeM27S II | 0.3 | 10 | 33 |

### Conclusions

- Several (methyl)lanthionine variants stimulate the cAMP pathway at concentrations lower or slightly higher than that of the linear S to A control.
- The capacity to stimulate the beta arrestin pathway is rather variable and depends on the peptide. This implies strongly differential activity of several variants; this is illustrated by the variation in the values of the last column which gives the ratio of both pathways.
- In particular lanthionine variants seem very active.
- Not only replacement of an amino acid by part of the lanthionine but also insertion of (part of) the lanthionine yields active variants.
- Two variants more effectively stimulate the beta arrestin pathway than the cAMP pathway.

## Claims

1. A cyclic apelin analog of the general formula X1-X2-X3X4-X5-X6-X7-X8-X9-X10-X11-X12-X13-X14, comprising one Lanthionine bridge of the structure Ala-S-Ala or one methylLanthionine bridge of the structure Abu-S-Ala or Ala-S-Abu, wherein
X1 is absent;
X2 is pGlu;
X3 is Arg
X4 is Pro, Pro-(me)Lan,;
X5 is Arg, (me)Lan, Arg-(me)Lan,;
X6 is Leu, (me)Lan, Leu-(me)Lan,;
X7 is Ala, (me)Lan,;
X8 is His, (me)Lan,;
X9 is Lys, (me)Lan, Lys-(me)Lan,;
X10 is Gly, (me)Lan,;
X11 is Pro, , (me)Lan, Pro-Lan;
X12 is Met, (me)Lan, Met-(me)Lan;
X13 is either absent or selected from Pro, Dhb, (me)Lan,;
X14 is the C-terminus and is either absent or is Phe, unless X13 is absent;
in which
Abu means aminobutyric acid and Dhb means dehydrobutyrine;
(me)Lan means Lan or meLan, wherein Lan denotes the N- or C-terminal half of a Lanthionine (Ala-S-Ala) and meLan denotes the N- or C-terminal half of a methylLanthionine (Abu-S-Ala or Ala-S-Abu);
with the proviso that:
(i) the analog contains up to two Ala residues;
(ii) the sequence X4 through X13 contains one pair of meLan or one pair of Lan which together form a (methyl)Lanthionine bridge; and
(iii) wherein said (methyl)Lanthionine bridge is of the size which comprises either two, three, four or five residues under the ring;
or an amide, an ester or a salt thereof.

2. Apelin analog according to claim 1, wherein X2-X3-X4-X5-X6 is pGlu-Arg-Pro-Arg-Leu.

3. Apelin analog according to any one of claims 1-2, wherein X5, X6, X7 or X9 is (me)Lan

4. Apelin analog according to any one of claims 1-3, wherein the sequence X7-X8-X9-X10 is (me)Lan-His-Lys-(me)Lan, preferably Lan-His-Lys-Lan, or wherein the sequence X7-X8-X9-X10 is (me)Lan-His-Lys-(me)Lan-Gly, preferably Lan-His-Lys-Lan-Gly.

5. Apelin analog according to claim 1, wherein the sequence X4-X5-X6-X7-X8 is Pro-(me)Lan-Arg-Leu-Ala-(me)Lan or Pro-Arg-(me)Lan-Arg Leu-Ala-(me)Lan.

6. Apelin analog according to any one of the preceding claims, wherein the sequence X11-X12-X13-X14 is (me)Lan-Met-Pro-Phe.

7. Apelin analog according to claim 1, selected from the group consisting of:
pERPmeLanRLAmeLanKGPMPF, pERPLanRLALanKGPMPF,
pERPRmeLanLAmeLanKGPMPF, pERPLanLAHLanGPMPF,
pERPRLmeLanHKmeLanPMPF, pERPRLmeLanHKmeLanPMdhbF,
pERPRLLanAHKLanPMPF, pERPRLLanAHKLanGPMPF,
pERPRLmeLanHKmeLanGPMPF, pERPRLmeLanHKmeLanGPMPF-NH2,
pERPRLmeLanHKmeLanGPMP, pERPRLLanHKLanGPMPF,
pERPRLmeLanHKGmeLanPMPF, pERPRLLanHKGLanPMPF,
pERPRLAHLanGPMLanF, pERPRLLanHKGLanMPF, pERPRLmeLanHKGmeLanMPF,
pERPRLLanHKGPLanPF, pERPRLLanHKGPLanMPF, pERPRLLanHKGPMLanF and
pERPRLLanHKGPMLanPF.

8. Apelin analog according to any one of claims 1-7, capable of inducing cAMP production in cells expressing an apelin (APJ) receptor with an EC50 of 0.1-200 nM preferably 0.1-5 nM.

9. Apelin analog according to any one of claims 1-8, exhibiting a reduced beta-arrestin associated internalization of the apelin receptor as compared to native apelin-13.

10. Apelin analog according to any one of claims 1-9, showing a stability (T50) in rat plasma of at least 3 hours, preferably at least 6 hours, more preferably at least 8 hours.

11. A pharmaceutical composition comprising at least one apelin analog according to any one of claims 1 to 10 for use as medicament.

12. Apelin analog according to any one of claims 1-11 for use in a method for treating a condition related to altered water balance, a stress-induced disorder such as anxiety and depression, a cardiovascular disorder or a metabolic disorder, preferably a cardiovascular disorder.

13. A method for providing an apelin analog according to any one of claims 1-11, comprising:
a) providing a host cell comprising:
- a nucleic acid molecule comprising a first nucleic acid fragment encoding an N-terminal leader peptide found within the precursor peptide of a lanthipeptide/lantibiotic and a second nucleic acid fragment encoding a peptide analog, whereby said first and second fragment are within the same open reading frame of said nucleic acid molecule;
- a nucleic acid sequence encoding an enzyme capable of dehydrating serine and/or threonine;
- optionally a nucleic acid sequence encoding a transporter protein;
b) allowing for the translation of said first nucleic acid; and
c) harvesting said peptide analog.

14. Method according to claim 13, comprising ring closure by chemical or enzymatic means, preferably by enzymatic means.

15. Method according to claim 13 or 14, wherein the second nucleic acid fragment encodes a polypeptide selected from the group consisting of QRPTRLACKGPMPF, QRPSRLACKGPMPF, QRPRTLACKGPMPF, QRPSLAHCGPMPF, QRPRLTHKCPMPF, QRPRLSAHKCPMPF, QRPRLSAHKCGPMPF, QRPRLTHKCGPMPF, QRPRLTHKCGPMPF-NH₂, QRPRLTHKCGPMP, QRPRLSHKCGPMPF, QRPRLTHKGCPMPF, QRPRLSHKGCPMPF, QRPRLAHSGPMCF, QRPRLSHKGCMPF, QRPRLTHKGCMPF, QRPRLSHKGPCPF, QRPRLSHKGPCMPF, QRPRLSHKGPMCF and QRPRLSHKGPMCPF.

## Patentansprüche

1. Zyklisches Apelin-Analogon der allgemeinen Formel X1-X2-X3-X4-X5-X6-X7-X8-X9-X10-X11-X12-X13-X14, das eine Lanthioninbrücke der Struktur Ala-S-Ala oder eine Methyllanthioninbrücke der Struktur Abu-S-Ala oder Ala-S-Abu umfasst, wobei
X1 fehlt;
X2 pGlu ist;
X3 Arg ist;
X4 Pro, Pro-(me)Lan ist;
X5 Arg, (me)Lan, Arg-(me)Lan ist;
X6 Leu, (me)Lan, Leu-(me)Lan ist;
X7 Ala, (me)Lan ist;
X8 His, (me)Lan ist;
X9 Lys, (me)Lan, Lys-(me)Lan ist;
X10 Gly, (me)Lan ist;
X11 Pro, (me)Lan, Pro-Lan ist;
X12 Met, (me)Lan, Met-(me)Lan ist;
X13 entweder fehlt oder aus Pro, Dhb, (me)Lan ausgewählt ist;
X14 der C-Terminus ist und entweder fehlt oder Phe ist, es sei denn, X13 fehlt;
wobei
Abu Aminobuttersäure bedeutet und Dhb Dehydrobutyrin bedeutet;
(me)Lan Lan oder meLan bedeutet, wobei Lan die N- oder C-terminale Hälfte eines Lanthionins (Ala-S-Ala) bezeichnet und meLan die N- oder C-terminale Hälfte eines Methyllanthionins (Abu-S-Ala oder Ala-S-Abu) bezeichnet;
mit der Maßgabe, dass:
(i) das Analogon bis zu zwei Ala-Reste enthält;
(ii) die Sequenz X4 bis X13 enthält ein Paar von meLan oder ein Paar von Lan enthält, die zusammen eine (Methyl)Lanthioninbrücke bilden; und
(iii) wobei die (Methyl)Lanthioninbrücke die Größe hat, die entweder zwei, drei, vier oder fünf Reste unter dem Ring enthält;
oder ein Amid, ein Ester oder ein Salz davon.

2. Apelin-Analogon nach Anspruch 1, wobei X2-X3-X4-X5-X6 pGlu-Arg-Pro-Arg-Leu ist.

3. Apelin-Analogon nach einem der Ansprüche 1-2, wobei X5, X6, X7 oder X9 (me)Lan ist.

4. Apelin-Analogon nach einem der Ansprüche 1-3, wobei die Sequenz X7-X8-X9-X10 (me)Lan-His-Lys-(me)Lan, vorzugsweise Lan-His-Lys-Lan, ist oder wobei die Sequenz X7-X8-X9-X10 (me)Lan-His-Lys-(me)Lan-Gly, vorzugsweise Lan-His-Lys-Lan-Gly, ist.

5. Apelin-Analogon nach Anspruch 1, wobei die Sequenz X4-X5-X6-X7-X8 Pro-(me)Lan-Arg-Leu-Ala-(me)Lan oder Pro-Arg-(me)Lan-Arg Leu-Ala-(me)Lan ist.

6. Apelin-Analogon nach einem der vorhergehenden Ansprüche, wobei die Sequenz X11-X12-X13-X14 (me)Lan-Met-Pro-Phe ist.

7. Apelin-Analogon nach Anspruch 1, ausgewählt aus der Gruppe, bestehend aus:
pERPmeLanRLAmeLanKGPMPF, pERPLanRLALanKGPMPF,
pERPRmeLanLAmeLanKGPMPF, pERPLanLAHLanGPMPF,
pERPRLmeLanHKmeLanPMPF,pERPRLmeLanHKmeLanPMdhbF,
pERPRLLanAHKLanPMPF, pERPRLLanAHKLanGPMPF,
pERPRLmeLanHKmeLanGPMPF, pERPRLmeLanHKmeLanGPMPF-NH2,
pERPRLmeLanHKmeLanGPMP. pERPRLLanHKLanGPMPF,
pERPRLmeLanHKGmeLanPMPF, pERPRLLanHKGLanPMPF,
pERPRLAHLanGPMLanF, pERPRLLanHKGLanMPF, pERPRLmeLanHKGmeLanMPF,
pERPRLLanHKGPLanPF, pERPRLLanHKGPLanMPF, pERPRLLanHKGPMLanF und
pERPRLLanHKGPMLanPF.

8. Apelin-Analogon nach einem der Ansprüche 1-7, das in der Lage ist, die cAMP-Produktion in Zellen, die einen Apelin(APJ)-Rezeptor exprimieren, mit einer EC50 von 0,1-200 nM, vorzugsweise 0,1-5 nM, zu induzieren.

9. Apelin-Analogon nach einem der Ansprüche 1-8, das eine verringerte beta-Arrestin-assoziierte Internalisierung des Apelin-Rezeptors im Vergleich zu nativem Apelin-13 zeigt.

10. Apelin-Analogon nach einem der Ansprüche 1-9, das eine Stabilität (T50) in Rattenplasma von mindestens 3 Stunden, vorzugsweise mindestens 6 Stunden, stärker bevorzugt mindestens 8 Stunden zeigt.

11. Pharmazeutische Zusammensetzung, die mindestens ein Apelin-Analogon nach einem der Ansprüche 1 bis 10 umfasst, zur Verwendung als Medikament.

12. Apelin-Analogon nach einem der Ansprüche 1-11 zur Verwendung bei einem Verfahren zur Behandlung eines Zustands, der durch einen veränderten Wasserhaushalt bedingt ist, einer durch Stress induzierten Störung, wie Angst und Depression, einer Herz-Kreislauf-Störung oder einer Stoffwechselstörung, vorzugsweise einer Herz-Kreislauf-Störung.

13. Verfahren zur Bereitstellung eines Apelin-Analogons nach einem der Ansprüche 1-11, das Folgendes umfasst:
a) Bereitstellen einer Wirtszelle, umfassend:
- ein Nukleinsäuremolekül, das ein erstes Nukleinsäurefragment, das ein N-terminales Leaderpeptid codiert, das man im Vorläuferpeptid eines Lanthipeptids/Lantibiotikums findet, und ein zweites Nukleinsäurefragment umfasst, das ein Peptidanalogon codiert, wobei das erste und das zweite Fragment sich innerhalb desselben offenen Leserahmens des Nukleinsäuremoleküls befinden;
- eine Nukleinsäuresequenz, die ein Enzym codiert, das zur Dehydratisierung von Serin und/oder Threonin in der Lage ist;
- gegebenenfalls eine Nukleinsäuresequenz, die ein Transporterprotein codiert;
b) Ermöglichen der Translation der ersten Nukleinsäure und
c) Ernten des Peptidanalogons.

14. Verfahren nach Anspruch 13, das einen Ringschluss durch chemische oder enzymatische Mittel, vorzugsweise durch enzymatische Mittel, umfasst.

15. Verfahren nach Anspruch 13 oder 14, wobei das zweite Nukleinsäurefragment ein Polypeptid codiert, ausgewählt aus der Gruppe, bestehend aus
QRPTRLACKGPMPF,
QRPSRLACKGPMPF, QRPRTLACKGPMPF, QRPSLAHCGPMPF, QRPRLTHKCPMPF, QRPRLSAHKCPMPF, QRPRLSAHKCGPMPF, QRPRLTHKCGPMPF, QRPRLTHKCGPMPF-NH₂, QRPRLTHKCGPMP, QRPRLSHKCGPMPF, QRPRLTHKGCPMPF, QRPRLSHKGCPMPF, QRPRLAHSGPMCF, QRPRLSHKGCMPF, QRPRLTHKGCMPF, QRPRLSHKGPCPF, QRPRLSHKGPCMPF, QRPRLSHKGPMCF und QRPRLSHKGPMCPF.

## Revendications

1. Analogue d'apéline cyclique de formule générale X1-X2-X3-X4-X5-X6-X7-X8-X9-X10-X11-X12-X13-X14, comprenant un pont lanthionine de structure Ala-S-Ala ou un pont méthyl-lanthionine bridge de structure Abu-S-Ala ou Ala-S-Abu, dans lequel
X1 est absent ;
X2 est pGlu ;
X3 est Arg
X4 est Pro, Pro-(me)Lan ;
X5 est Arg, (me)Lan, Arg-(me)Lan ;
X6 est Leu, (me)Lan, Leu-(me)Lan ;
X7 est Ala, (me)Lan ;
X8 est His, (me)Lan ;
X9 est Lys, (me)Lan, Lys-(me)Lan ;
X10 est Gly, (me)Lan ;
X11 est Pro, (me)Lan, Pro-Lan ;
X12 est Met, (me)Lan, Met-(me)Lan ;
X13 est absent ou choisi parmi Pro, Dhb, (me)Lan ;
X14 est l'extrémité C-terminale et est absent ou est Phe, sauf si X13 est absent ;
dans lequel
Abu désigne l'acide aminobutyrique et Dhb désigne la déshydrobutyrine ;
(me)Lan désigne Lan ou meLan, dans lequel Lan désigne la moitié N- ou C-terminale d'une lanthionine (Ala-S-Ala) et meLan désigne la moitié N- ou C-terminale d'une méthyl-lanthionine (Abu-S-Ala ou Ala-S-Abu) ;
à condition que :
(i) l'analogue contienne jusqu'à deux résidus Ala ;
(ii) la séquence X4 à X13 contienne une paire de meLan ou une paire de Lan qui forment conjointement un pont (méthyl)lanthionine ; et
(iii) dans lequel ledit pont (méthyl)lanthionine est de la taille qui comprend deux, trois, quatre ou cinq résidus sous le cycle ;
ou amide, ester ou sel de celui-ci.

2. Analogue d'apéline selon la revendication 1, dans lequel X2-X3-X4-X5-X6 est pGlu-Arg-Pro-Arg-Leu.

3. Analogue d'apéline selon l'une quelconque des revendications 1 à 2, dans lequel X5, X6, X7 ou X9 est (me)Lan.

4. Analogue d'apéline selon l'une quelconque des revendications 1 à 3, dans lequel la séquence X7-X8-X9-X10 est (me)Lan-His-Lys-(me)Lan, de préférence Lan-His-Lys-Lan, ou dans lequel la séquence X7-X8-X9-X10 est (me)Lan-His-Lys-(me)Lan-Gly, de préférence Lan-His-Lys-Lan-Gly.

5. Analogue d'apéline selon la revendication 1, dans lequel la séquence X4-X5-X6-X7-X8 est Pro-(me)Lan-Arg-Leu-Ala-(me)Lan ou Pro-Arg-(me)Lan-Arg Leu-Ala-(me)Lan.

6. Analogue d'apéline selon l'une quelconque des revendications précédentes, dans lequel la séquence X11-X12-X13-X14 est (me)Lan-Met-Pro-Phe.

7. Analogue d'apéline selon la revendication 1, choisi dans le groupe constitué de :
pERPmeLanRLAmeLanKGPMPF, pERPLanRLALanKGPMPF,
pERPRmeLanLAmeLanKGPMPF, pERPLanLAHLanGPMPF,
pERPRLmeLanHKmeLanPMPF, pERPRLmeLanHKmeLanPMdhbF,
pERPRLLanAHKLanPMPF, pERPRLLanAHKLanGPMPF,
pERPRLmeLanHKmeLanGPMPF, pERPRLmeLanHKmeLanGPMPF-NH2,
pERPRLmeLanHKmeLanGPMP, pERPRLLanHKLanGPMPF,
pERPRLmeLanHKGmeLanPMPF, pERPRLLanHKGLanPMPF,
pERPRLAHLanGPMLanF, pERPRLLanHKGLanMPF,
pERPRLmeLanHKGmeLanMPF, pERPRLLanHKGPLanPF,
pERPRLLanHKGPLanMPF, pERPRLLanHKGPMLanF et
pERPRLLanHKGPMLanPF.

8. Analogue d'apéline selon l'une quelconque des revendications 1 à 7, capable d'induire la production d'AMPc dans des cellules exprimant un récepteur d'apéline (APJ) ayant une CE50 de 0,1 à 200 nM, de préférence 0,1 à 5 nM.

9. Analogue d'apéline selon l'une quelconque des revendications 1 à 8, présentant une internalisation associée à la bêta-arrestine réduite par rapport à l'apéline 13 native.

10. Analogue d'apéline selon l'une quelconque des revendications 1 à 9, présentant une stabilité (T50) dans le plasma de rat d'au moins 3 heures, de préférence au moins 6 heures, plus préférablement au moins 8 heures.

11. Composition pharmaceutique comprenant au moins un analogue d'apéline selon l'une quelconque des revendications 1 à 10, pour utilisation en tant que médicament.

12. Analogue d'apéline selon l'une quelconque des revendications 1 à 11, pour utilisation dans un procédé de traitement d'une affection liée à un bilan hydrique altéré, un trouble induit par le stress tel que l'anxiété et la dépression, un trouble cardiovasculaire ou un trouble métabolique, de préférence un trouble cardiovasculaire.

13. Procédé de fourniture d'un analogue d'apéline selon l'une quelconque des revendications 1 à 11, comprenant :
a) la fourniture d'une cellule hôte comprenant :
- une molécule d'acide nucléique comprenant un premier fragment d'acide nucléique codant pour un peptide leader N-terminal d'ADNc présent dans le peptide précurseur d'un lanthipeptide/lantibiotique et un deuxième fragment d'acide nucléique codant pour un analogue de peptide, lesdits premier et deuxième fragments sont dans le même cadre ouvert de lecture de ladite molécule d'acide nucléique ;
- une séquence d'acide nucléique codant pour une enzyme capable de déshydrater la sérine et/ou la thréonine ;
- facultativement une séquence d'acide nucléique codant pour une protéine de transporteur ;
b) la conduite de la traduction dudit premier acide nucléique ; et
c) la collecte dudit analogue de peptide.

14. Procédé selon la revendication 13, comprenant une fermeture de cycle par des moyens chimiques ou enzymatiques, de préférence par des moyens enzymatiques.

15. Procédé selon la revendication 13 ou 14, dans lequel le deuxième fragment d'acide nucléique code pour un polypeptide choisi dans le groupe constitué de QRPTRLACKGPMPF, QRPSRLACKGPMPF, QRPRTLACKGPMPF, QRPSLAHCGPMPF, QRPRLTHKCPMPF, QRPRLSAHKCPMPF, QRPRLSAHKCGPMPF, QRPRLTHKCGPMPF, QRPRLTHKCGPMPF-NH₂, QRPRLTHKCGPMP, QRPRLSHKCGPMPF, QRPRLTHKGCPMPF, QRPRLSHKGCPMPF, QRPRLAHSGPMCF, QRPRLSHKGCMPF, QRPRLTHKGCMPF, QRPRLSHKGPCPF, QRPRLSHKGPCMPF, QRPRLSHKGPMCF et QRPRLSHKGPMCPF.
